# EUROPEAN PATENT APPLICATION

(11) **EP 3 202 362 A1**
(43) Date of publication of application: **09.08.2017**
(21) Application number: 14903137.9
(22) Date of filing: 30.09.2014
(51) Int. Cl.: A61B 34/00

(54) **ADAPTER COMPONENT**

(71) Applicant: Riverfield Inc., Shinjuku-ku Tokyo 160-0017 (JP)
(72) Inventor: HARAGUCHI, Daisuke, Tokyo 160-0023 (JP); MIKAMI, Kei, Tokyo 160-0023 (JP)
(74) Representative: chapman + co
(86) International application number: PCT/JP2014/076060
(87) International publication number: WO 2016/051494

(57) **Abstract**

In order to reliably separate an unsterilized zone from a sterilized zone in a device for connecting a medical instrument, an adapter component set (28) is for connecting the medical instrument such as an endoscope to an unsterilized device having a timing pulley (27) included in a rotation mechanism for rotating the medical instrument. An adapter (47) holds the medical instrument. A separator (46) has a hollow cylinder shape for inserting the adapter therein. At least the inner surface of the separator is included in the sterilized zone, and the outer surface of the cylinder is connected to the pulley. As connecting parts for connecting a drape for isolating the device having the rotation mechanism from the sterilized zone, a pair of an outlet drape bearing (41) and an outlet drape bearing cover (42) and a pair of an inlet drape bearing (45) and an inlet drape bearing cover (44) are provided.

## Description

### TECHNICAL FIELD

The present invention relates to an adapter component set suitable to be used as a device for connecting a medical instrument.

### BACKGROUND ART

Conventionally, in a surgical operation, an endoscopic operation has been broadly carried out instead of an abdominal operation because the endoscopic operation has an advantage of a rapid speed of a postoperative recovery, an advantage of having a small wound in the operation, and so on. In such an endoscopic operation, an endoscopic operation system, which uses a robot arm to hold and to operate the endoscope, has been proposed (for example, refer to a patent document 1).

In the system, the endoscopic operation system of an unsterilized zone has to be separated from a sterilized zone where all parts are sterilized in order to keep the whole space where the patient is positioned as the sterilized zone. For this reason, conventionally, a sterile surgical operation adapter (refer to a patent document 2), a sterilization drape for a surgery operation (refer to a patent document 3), and so on have been used.

### PRIOR ART DOCUMENT

### PATENT DOCUMENT

Patent document 1: JP2014-094039, A
Patent document 2: JP2007-167644, A
Patent document 3: JP2009-509653, A

### SUMMARY OF THE INVENTION

### PROBLEMS TO BE SOLVED BY THE INVENTION

However, it has been difficult to reliably separate the endoscopic operation system in an unsterilized zone from a sterilized zone by using conventional skill including skill described in the patent documents 1 to 3.

This applies to not only an endoscope but also any device that connects a medical instrument.

The present invention is created in view of such a situation. It is an object of the present invention to reliably separate an unsterilized zone and a sterilized zone from each other in a device for connecting a medical instrument thereto.

### MEANS FOR SOLVING THE PROBLEM

An adapter component set in one aspect of the present invention is characterized by the following:
an adapter component set for connecting a medical instrument to a device that is included in an unsterilized zone and has a rotation mechanism for rotating the medical instrument, comprising:
   an adapter for holding the medical instrument;
   a separator having a shape of a hollow cylinder into which the adapter is inserted, at least an inner surface of the separator being included in a sterilized zone, and an outer surface of the separator being configured to be capable of being connected to the rotation mechanism; and
   connecting parts to which a drape is connected, the drape separating the device having the rotation mechanism from the sterilized zone.

First engagement parts for engaging the adapter and the separator, and second engagement parts for engaging the separator and the rotation mechanism may be further provided.

In this case, an engagement strength of the first engagement parts may be set to be weaker than an engagement strength of the second engagement parts.

### EFFECT OF THE INVENTION

According to the present invention, an unsterilized zone and a sterilized zone can be reliably separated from each other in a device for connecting a medical instrument thereto.

### BRIEF DESCRIPTION OF THE DRAWINGS

FIG. 1 is a perspective view showing schematic outer construction of a robot arm in a robot system according to an embodiment of the present invention;
FIG. 2 is an enlarged partial longitudinal sectional view showing an adapter component set and the neighborhood, of the robot arm in FIG. 1;
FIG. 3 is a side view showing an outer construction of the adapter component set, which a surgical instrument has been attached to, of the robot arm in FIG. 1;
Fig. 4 is a perspective view showing an outer construction of the adapter component set in a state where respective components are separated from each other;
FIGS. 5A, 5B, 5C are views showing outer constructions of the adapter component set in a state where the respective components shown in FIG. 4 are assembled;
FIGS. 6A, 6B, 6C are views of the adapter component set for explaining a state where an adapter is inserted into a separator;
FIGS. 7A, 7B are views of the adapter component set for explaining a state where a timing pulley, the separator, and the adapter have been assembled;
FIG. 8 is a block diagram showing a hardware construction to carry out a pneumatic servo control for the robot arm in the robot system according to the embodiment of the present invention;
FIG. 9 is a functional block diagram showing a schematic control system of the pneumatic servo control for the robot arm, which servo control is carried out by the hardware construction shown in FIG. 8;
FIG. 10 is a flow chart showing a processing of the pneumatic servo control to be carried out by a control section in FIG. 8, which control section works according to the control system shown in FIG. 9.

### MODE FOR CARRYING OUT THE INVENTION

An embodiment of the present invention will be described with reference to the attached drawings below.

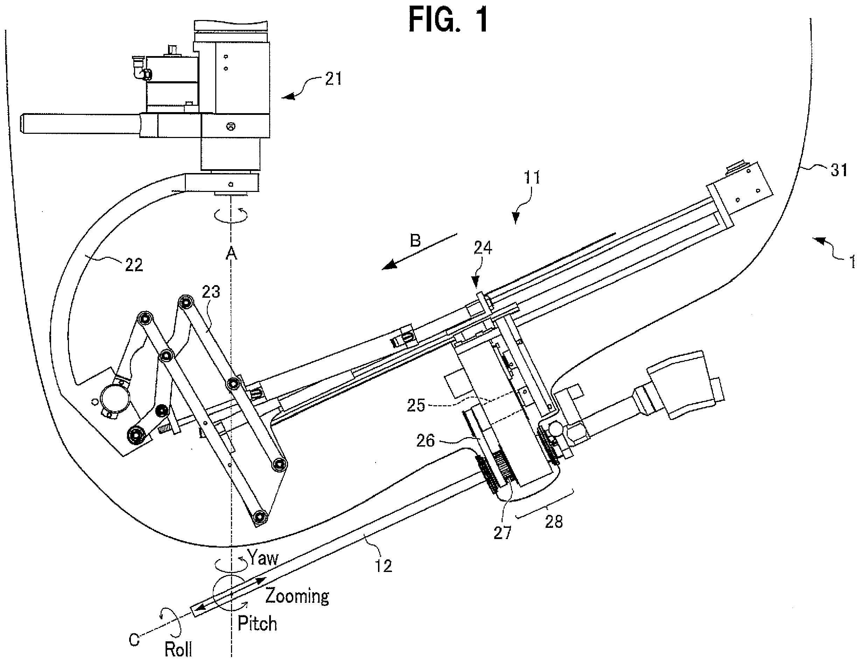

Figure 1 is a perspective view showing schematic outer construction of a robot arm 11 in a robot system 1 according to the embodiment of the present invention.

The robot system 1 of the present embodiment has the robot arm 11 driven by a pneumatic system, which arm can hold and operate a surgical instrument 12 such as an endoscope, a forceps, etc.

The robot arm 11 is driven by a pneumatic servo control, and has a passive operation mode and an active operation mode in which the robot arm operates in different ways.

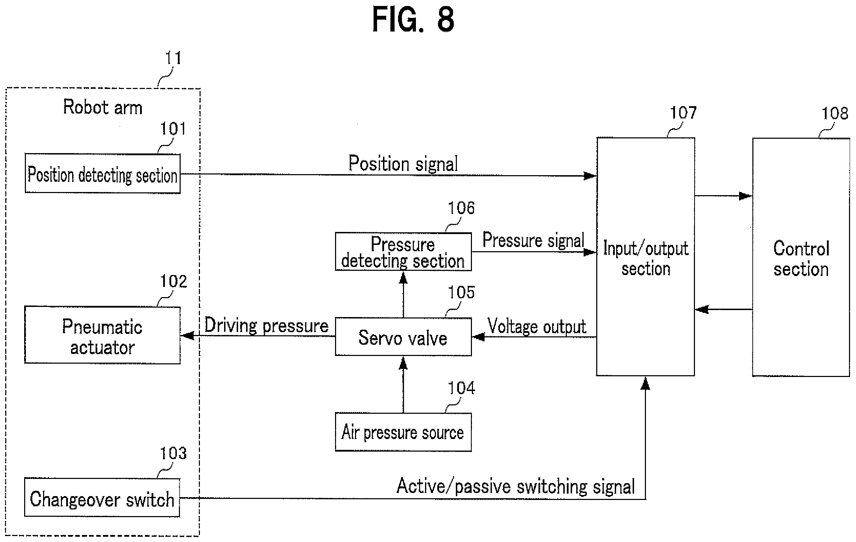

The passive operation mode is a mode which functions while a user (a doctor to operate, etc.) keeps depressing of a switch (a changeover switch 103 shown in Figure 8 to be described later) in the present embodiment. This mode is a mode in which the user moves the robot arm 11 as if he moves it by manual operation with an intuitive feeling similar to that in a usual case of his holding of a surgical instrument such as the endoscope, etc. In actual, in this mode, a self weight compensation control and a pressure control are carried out.

The active operation mode is a mode in which the robot arm 11 is automatically moved to a designated target position by an automatic control having a position control and a pressure control.

The robot arm 11 has a front end part of the surgical instrument 12 so as to be able to move with 4 degrees of freedom. That is, the robot arm 11 holds the surgical instrument 12 so that at an intersection point of an axis A and an axis C, it can be moved in a direction of rotation (roll) around the axis C, in a direction of rotation (yaw) around the axis A, in a direction of rotation (pitch) caused by a parallel driving mechanism 23, and in a direction approximately parallel with a direction B.

Specifically, the robot arm 11 has a rotation driving device 21, a connecting member 22, the parallel driving mechanism 23, a pneumatic cylinder 24, a rotation motor 25, a timing belt 26, a timing pulley 27, and an adapter component set 28.

The rotation driving device 21 rotates the surgical instrument 12 around the yaw axis by rotating the robot arm 11 around the axis A.

The connecting member 22 connects the parallel driving mechanism 23 with the rotation driving device 21 so that it is moved by following a rotation around the axis A.

The parallel driving mechanism 23 makes the surgical instrument 12 rotate in the direction of pitch rotation by rotating the robot arm 11.

The pneumatic cylinder 24 is a pneumatic cylinder to drive the robot arm 11 in the direction approximately parallel with the direction B.

The rotation motor 25 generates a rotational force (torque), and transmits the rotational force to the timing pulley 27 via the timing belt 26.

The timing belt 26 is a belt for connecting the rotation motor 25 with the timing pulley 27.

The timing pulley 27 makes the surgical instrument 12 set in the adapter component set 28 rotate in the direction of roll rotation by the rotational force transmitted via the timing belt 26 from the rotation motor 25.

The adapter component set 28 is a component set which includes the timing pulley 27 and rotatably attaches the surgical instrument 12 to the robot arm 11.

The robot arm 11 constructed like that is a device in an unsterilized zone.

Therefore, as shown in Figure 1, the robot arm 11 in the unsterilized zone is separated from a sterilized zone in which a patient during surgery is positioned by covering the robot arm 11 with a drape 31.

However, the surgical instrument 12 is necessary to be positioned in the sterilized zone, and is also necessary to be rotatably supported by the adapter component set 28. Therefore, if any treatment is not done, there is also a fear that the surgical instrument 12 comes into contact with the unsterilized zone.

Then, ingenuities that the unsterilized zone and the sterilized zone are perfectly separated from each other are included in the adapter component set 28 in the present embodiment.

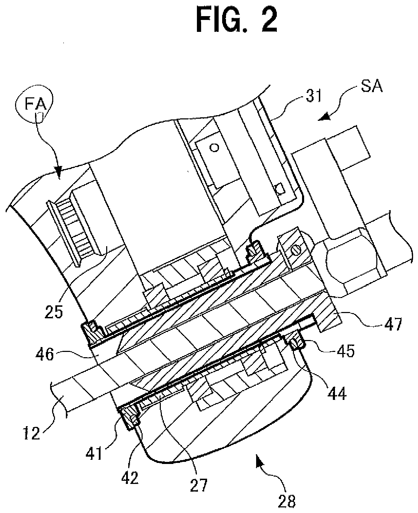

Hereinafter, such adapter component set 28 will be specifically described with reference to Figures 2 to 7.

Figure 2 is an enlarged partial longitudinal sectional view showing the adapter component set 28 and the neighborhood of the robot arm shown in Figure 1.

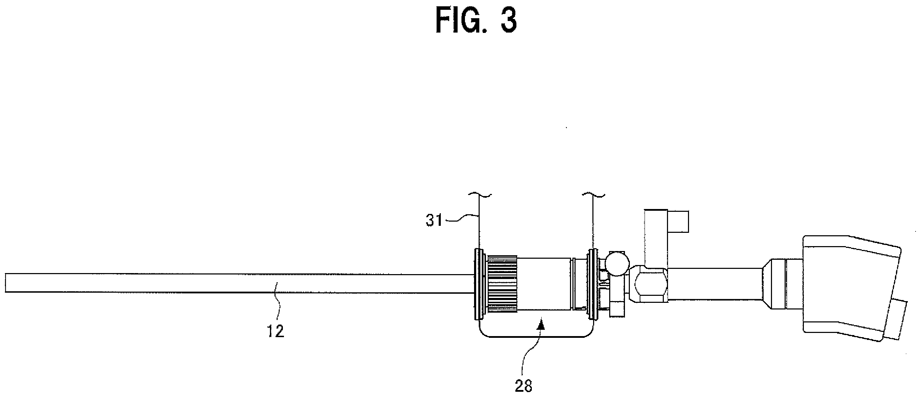

Figure 3 is a side view showing an outer construction of the adapter component set 28, which a surgical instrument has been attached to, of the robot arm 11 shown in Figure 1.

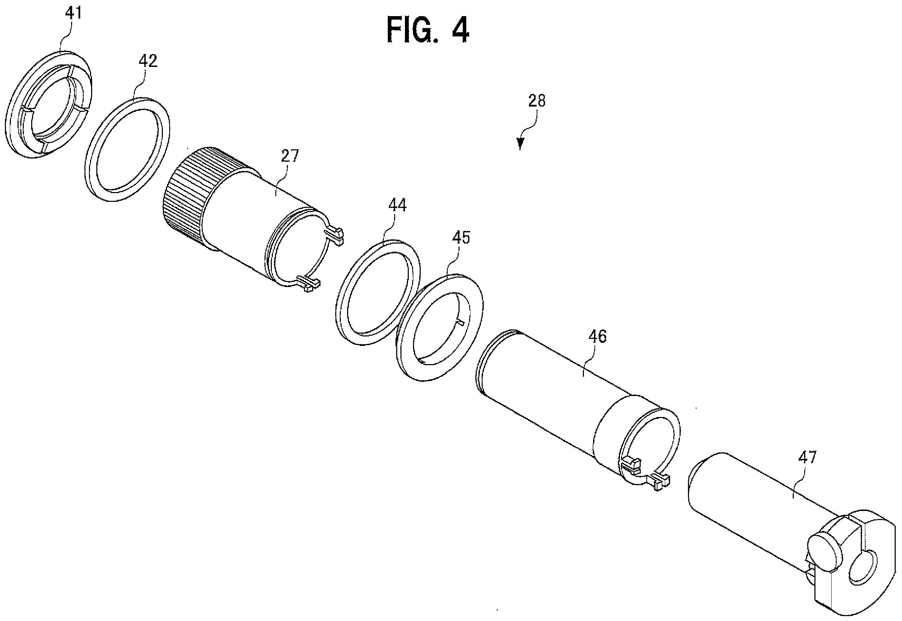

Figure 4 is a perspective view showing an outer construction of the adapter component set 28 in a state where respective components are separated from each other.

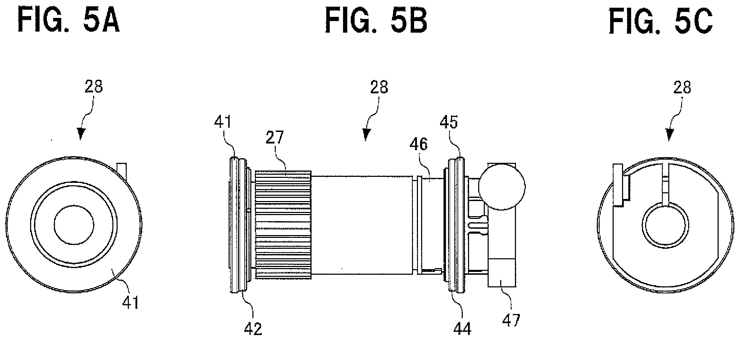

Figures 5A, 5B, 5C are views showing outer constructions of the adapter component set 28 in a state where the respective components shown in Figure 4 are assembled. Hereinafter, a front end side (left end side in Figure 3) of the surgical instrument 12 is called as an "outlet side or outlet". And the opposite side (right end side in Figure 3) is called as an "inlet side or inlet". Figure 5A is a front view of the outlet side of the adapter component set 28. Figure 5B is a side view of the adapter component set 28. Figure 5C is a front view of the inlet side of the adapter component set 28.

As shown in Figures 2 to 5 (especially in Figure 4), when viewed from the outlet side of the surgical instrument 12, the adapter component set 28 is constructed with an outlet drape bearing 41, an outlet drape bearing cover 42, the foresaid timing pulley 27, an inlet drape bearing cover 44, an inlet drape bearing 45, a separator 46, and an adapter 47, which are assembled.

In the drape 31 shown in Figure 3 and so on, two holes are provided, each of which has a diameter a little larger than a diameter of the separator 46. As shown in Figure 2, a peripheral region of one of the two holes is held between the outlet drape bearing 41 and the outlet drape bearing cover 42, and a peripheral region of the other hole is held between the inlet drape bearing cover 44 and the inlet drape bearing 45.

In this way, as shown in Figures 1 to 3, the robot arm 11 is covered with the drape 31. That is, basically, the unsterilized zone FA of the robot arm 11 is separated from the sterilized zone SA.

However, the surgical instrument 12 to be positioned in the sterilized zone SA may come into contact with the unsterilized zone FA in the adapter component set 28. Because of this, in the present embodiment, an inner surface of the separator 46 is included in the sterilized zone SA.

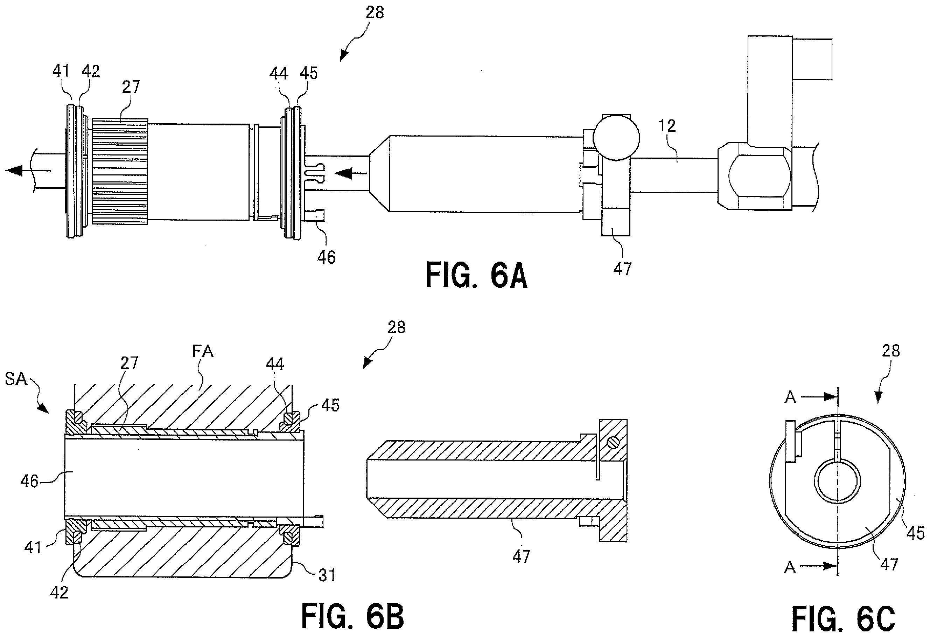

Hereby, the unsterilized zone FA can be perfectly separated from the sterilized zone SA with a boundary of the inner surface of the separator 46 and the drape 31 (that is, with a bold line in Figure 2 as a boundary line). Moreover, this will be described in detail with reference to Figure 6.

Figures 6A, 6B, 6C show the adapter component set 28 having the adapter 47 being inserted in the separator 46.

Specifically, Figure 6A is a side view of the adapter component set 28. Figure 6B is a longitudinal sectional view of the adapter component set 28. Figure 6C is a front view of the inlet side of the adapter component set 28. Figure 6B is the longitudinal sectional view taken along a line A in Figure 6C.

As shown in Figures 6 A, 6B, 6C, the adapter 47 which is set with the surgical instrument 12, is inserted into the inside of the separator 46 from the inlet side to the outlet side. At the time of such insertion of the adapter 47, there is a possibility that the surgical instrument 12 comes into contact with the inside of the separator 46. Therefore, the inside of the separator 46 must not be included in the unsterilized zone FA.

For this reason, at least the inner surface of the separator 46 is a sterilized part included in a sterilized zone where all parts are sterilized as described above. Hereby, the adapter 47 can be inserted into the separator 46 without the surgical instrument 12 coming into contact with the unsterilized zone FA.

Furthermore, the adapter 47 is also a sterilized part. Therefore, as shown in Figure 2, also after the adapter 47 is inserted into the separator 46, the unsterilized zone FA is perfectly separated from the sterilized zone SA with a boundary of the inner surface of the separator 46 and the drape 31 (with the bold line in Figure 2 as a boundary line).

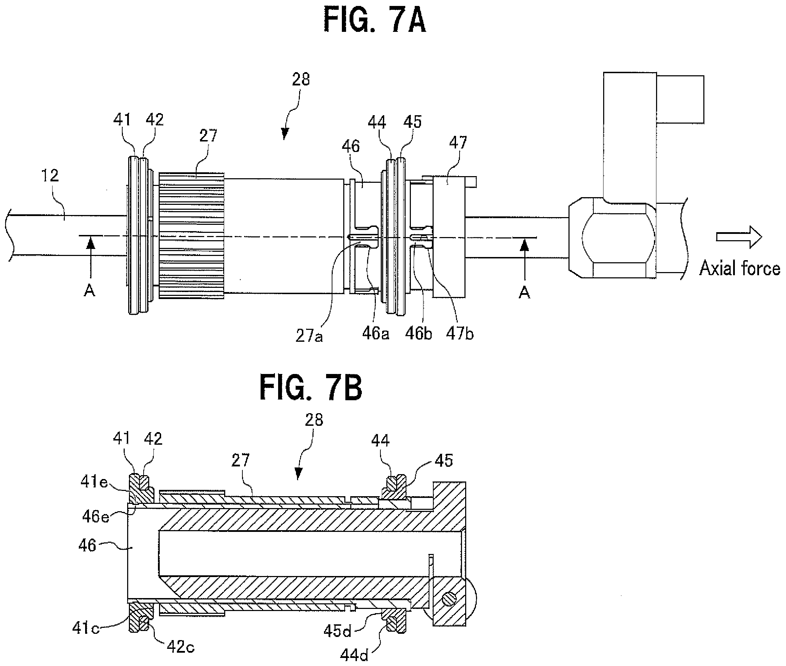

Furthermore, since the surgical instrument 12 of the present embodiment is rotated about its axis (rotated around an axis C in Figure 1), the rotational force of the rotation motor 25 is necessary to be transmitted to the surgical instrument 12 via the timing pulley 27. The structure for this purpose will be described with reference to Figures 7A, 7B.

Figures 7A, 7B show the adapter component set 28 having the timing pulley 27, the separator 46 and the adapter 47, which are assembled.

Specifically, Figure 7A is a side view of the adapter component set 28. Figure 7B is a longitudinal sectional view of the adapter component set 28. Figure 7B is a view taken along a line A in Figure 7A.

As shown in Figures 7A, 7B, the timing pulley 27 has a male engagement part 27a on the inlet side thereof. The separator 46 has a female engagement part 46a to be engaged with the male engagement part 27a when the separator 46 is inserted into the timing pulley 27.

That is, the timing pulley 27 and the separator 46 are joined by the engagement of the male engagement part 27a and the female engagement part 46a.

As shown in Figures 7A, 7B, the adapter 47 has a female engagement part 47b on the inlet side thereof. The separator 46 has a male engagement part 46b to be engaged with the female engagement part 47b when the adapter 47 is inserted into the separator.

That is, the separator 46 and the adapter 47 are joined by the engagement of the female engagement part 47b and the male engagement part 46b.

In the present embodiment, the engagement strength of the separator 46 and the adapter 47 (the engagement strength of the female engagement part 47b and the male engagement part 46b) is weaker than that of the timing pulley 27 and the separator 46 (the engagement strength of the male engagement part 27a and the female engagement part 46a).

Hereby, the joining of the separator 46 and the adapter 47 is disconnected earlier when a force in the axial direction (refer to Figure 7A) of the surgical instrument is applied to the surgical instrument 12.

Consequently, the risk of pushing the surgical instrument 12 is reduced and the unsterilized zone FA can be reliably prevented from being exposed in the sterilized zone SA.

Furthermore, an engagement part 41C and an engagement part 42C are provided also at joining parts of the outlet drape bearing 41 and the outlet drape bearing cover 42, respectively. That is, the outlet drape bearing 41 does not come off because of the engaging construction of the engagement part 41C and the engagement part 42C. Furthermore, an engagement part 41e and an engagement part 46e are provided also at joining parts of an inside and so on of the outlet drape bearing 41 and the separator 46, respectively. That is, the outlet drape bearing 41 and the separator 46 are engaged by the engaging construction of the engagement part 41e and the engagement part 46e. Thus, fixing of an insertion hole of the drape 31 can be done.

Similarly, an engagement part 45d and an engagement part 44d are provided also at joining parts of the inlet drape bearing 45 and the inlet drape bearing cover 44, respectively. That is, the inlet drape bearing 45 is configured not to come off because of the engaging construction of the engagement part 45d and the engagement part 44d.

This embodiment ensures that the unsterilized zone FA can be reliably prevented from being exposed in the sterilized zone SA on account of coming off of the drape 31.

As described above, the adapter component set 28 includes:
the separator 46 which is connected with the timing pulley 27 included in a rotation mechanism of the robot arm 11 in the unsterilized zone and has the hollow cylinder shape, the inner surface of the separator being configured to be included in the sterilized zone;
the adapter 47 which the surgical instrument 12 is capable of being attached to, and is inserted in the hole of the hollow cylinder of the separator 46; and
the outlet drape bearing 41 and the inlet drape bearing 45 for attaching the drape 31 for covering the robot arm 11 including the timing pulley 27 to isolate the robot arm from the sterilized zone.

Hereby, the unsterilized zone and the sterilized zone can be perfectly separated from each other.

Furthermore, the female engagement part 47b and the male engagement part 46b as engagement parts are provided in order to join the separator 46 and the adapter 47 to transmit a rotational force and to fix the surgical instrument 12.

Also with regard to the separator 46 and the timing pulley 27 included in the rotation mechanism of the robot arm 11, the male engagement part 27a and the female engagement part 46a are provided as engagement parts to transmit the rotational force.

In the present embodiment, the strength of the engagement between the separator 46 and the adapter 47 is configured to be weaker than that between the separator 46 and the robot arm 11. Hereby, a function that the adapter 47 is disengaged from the separator 46 when a pushing force over the predetermined force strength is applied to the surgical instrument 12, is realized.

Next, a pneumatic servo control for the robot arm 11 will be described with reference to Figures 8 to 10.

Figure 8 is a block diagram showing a hardware construction to carry out the pneumatic servo control for the robot arm 11 in a robot system according to an embodiment of the present invention.

As shown in Figure 8, in order to carry out the pneumatic servo control for the robot arm 11, an air pressure source 104, a servo valve 105, a pressure detecting section 106, an input/output section 107, and a control section 108 are provided.

The robot arm 11 is provided with a position detecting section 101, a pneumatic actuator 102 and a changeover switch 103.

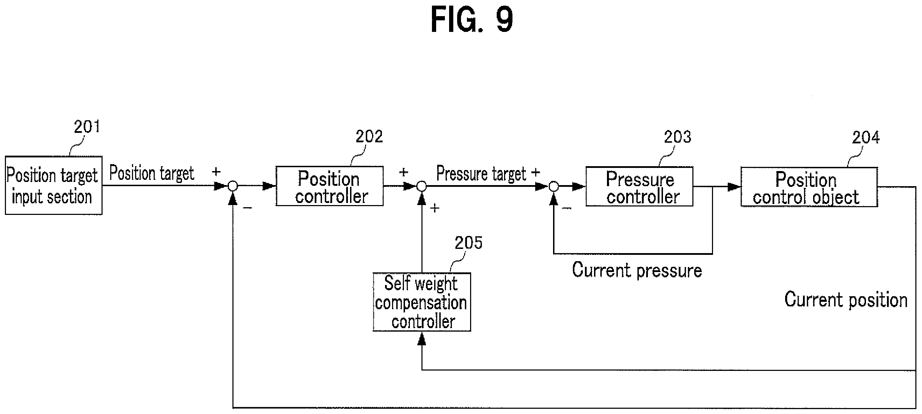

Figure 9 is a functional block diagram showing a schematic control system of the pneumatic servo control for the robot arm 11, which servo control is carried out by the hardware construction shown in Figure 8.

In the present embodiment, each functional block in Figure 9 is provided in the control section 108 in Figure 8. Note that, each functional block may be constituted by only hardware, by only software, or by a combination of software and hardware.

In the control system shown in Figure 9, a position target input section 201 outputs a position target.

A difference (error) between the position target and a current position output from a position control object 204 is input to a position controller 202. The position controller 202 carries out the position control so as to decrease the difference to zero, so that the position controller 202 outputs a pressure target.

The position control object 204 is the robot arm 11 in the present embodiment. Because of this, a position signal output from the position detecting section 101 provided in the robot arm 11 is input to the control section 108 via the input/output section 107. This position signal or a signal processed from the position signal is used as the current position in Figure 9.

In this way, the position control constitutes a main loop system of the control system in Figure 9.

Furthermore, the pressure control constitutes a sub loop system of the control system in Figure 9.

As described above, the passive operation mode and the active operation mode can be selectively used in the present embodiment.

At the time of the active operation mode, an output from the position controller 202 is input to a pressure controller 203 as a pressure target.

On the other hand, at the time of the passive operation mode, an output from the position controller 202 is inhibited (an output is zero), and an output from a self weight compensation controller 205 is input to the pressure controller 203 as a pressure target. That is, the self weight compensation controller 205 compensates a self weight based on the current position, and output the compensated result as the pressure target.

A difference (error) between the pressure target and a current pressure is input to the pressure controller 203. The pressure controller 203 carries out the pressure control so as to decrease the difference to zero.

A controlled result of the pressure controller 203, that is, an output from the pressure controller 203 is supplied to the position control object 204. The position control object 204 is the robot arm 11 in the present embodiment.

Specifically, the output from the pressure controller 203 is input to the servo valve 105 via the input/output section 107 as a voltage output.

The servo valve 105 supplies the air in the air pressure source 104 having a pressure corresponding to the voltage output, to the pneumatic actuator 102. That is, the pressure corresponding to the voltage output is a driving pressure, and the pneumatic actuator 102 is driven according to the driving pressure. Typically, the pneumatic actuator 102 is the pneumatic cylinder 24 in Figure 1. However, the pneumatic actuator 102 is not limited to this, and includes all that drive the robot arm 11 by means of air pressure.

The driving pressure is detected by the pressure detecting section 106, and is input to the control section 108 via the input/output section 107 as a pressure signal. This pressure signal or a signal processed from the pressure signal is used as the current pressure in Figure 9.

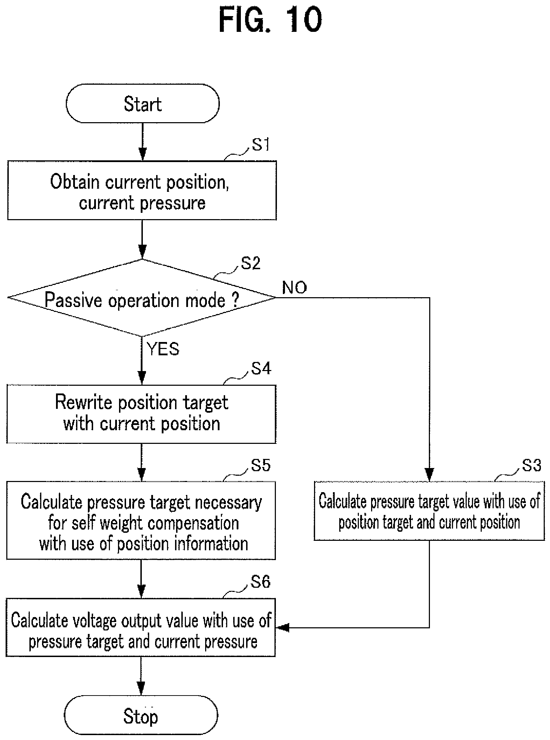

Figure 10 is a flow chart showing a processing of the pneumatic servo control to be carried out by the control section 108 in Figure 8, which control section works according to the control system shown in Figure 9.

In step S1, the control section 108 obtains a current position and a current pressure.

As described above, the control section 108 obtains a position signal output from the position detecting section 101 via the input/output section 107 as a current position, and furthermore, obtains a pressure signal output from the pressure detecting section 106 via the input/output section 107 as a current pressure.

In step S2, the control section 108 determines whether the operation mode is set to a passive operation mode or not.

In the present embodiment, we assume that when the changeover switch 103 in Figure 8 is in a state of keeping depressing of the switch, the operation mode is a passive operation mode, and when the changeover switch 103 is in the other state, the operation mode is an active operation mode.

The passive operation mode is a mode in which the surgical instrument 12 is operated as if a user (doctor etc.) operates it by manual operation. Therefore, it is preferable that the changeover switch 103 is provided near a head (the end opposite to the front end, that is, an inlet end) of the surgical instrument 12.

When the changeover switch 103 is not depressed, in step S2, the determination that the judgment result is "NO" is made. That is, the determination that the operation mode is the active operation mode, is made, and then the processing proceeds to step S3.

In step S3, the control section 108 calculates a pressure target value with use of the position target and the current position.

In step S6, the control section 108 calculates a voltage output value with use of the pressure target value and the current pressure.

The voltage output value is input to the servo valve 105 via the input/output section 107. The servo valve 105 supplies the air in the air pressure source 104 having a driving pressure
corresponding to the voltage output value, to the pneumatic actuator 102.

In this way, the driving control of the pneumatic actuator 102 according to the active operation mode is realized.

In contrast, when the changeover switch 103 is depressed, in step S2, the determination that the judgment result is "YES" is made. That is, the determination that the operation mode is the passive operation mode is made, and then the processing proceeds to step S4.

In step S4, the control section 108 rewrites the position target with the current position. That is, the error becomes zero, so the control is carried out so that an output from the position controller 202 in Figure 9 becomes zero, in other words, the control is carried out so that the pressure difference becomes zero.

In step S5, the control section 108 calculates a pressure target value (pitch axis) necessary for the self weight compensation with use of the position information (current position). That is, the self weight compensation controller 205 in Figure 9 calculates the pressure target value (pitch axis).

In step S6, the control section 108 calculates a voltage output value with use of the pressure target and the current pressure.

The voltage output value is input to the servo valve 105 via the input/output section 107. The servo valve 105 supplies the air in the air pressure source 104 having a driving pressure
corresponding to the voltage output value, to the pneumatic actuator 102.

In this way, the driving control of the pneumatic actuator 102 according to the passive operation mode is realized.

As described above, the robot arm 11 which is a pneumatic control object of the present embodiment is a robot arm which holds the surgical instrument 12 such as an endoscope, has 4 degrees of freedom and is operated by pneumatic drive.

We assume that the changeover switch 103 is provided near the head of the surgical instrument 12 (endoscope head) in a state where the surgical instrument 12 is set to the robot arm 11, in the present embodiment. Hereby, the user (doctor etc.) can change the operation mode to the passive operation mode only by depressing the changeover switch 103. During the passive operation mode, he can move the surgical instrument 12 set to the robot arm 11 with a feeling similar to that in the case of smoothly moving a conventional endoscope by his own hand.

In order to realize this smooth movement, in the present embodiment, the pneumatic actuator 102 is operated according to the pneumatic servo control with use of the pneumatic servo valve 105. That is, the pneumatic servo control is carried out according to an external force following control having a pressure feedback. In the passive operation mode, a light and smooth operation can be carried out, especially by compensating the self weight in the pressure feedback control for the pneumatic actuator 102.

Furthermore, in the case where the changeover switch 103 is released from the depressed state, the operation mode becomes the active operation mode. Therefore, the automatic position control can be carried out.

Note that, the present invention is not limited to the present embodiment, and modifications or improvements are included in the present invention within the range of achieving the object of the present invention.

For example, in the above-described embodiment, the surgical instrument 12 such as an endoscope, a forceps, etc. is set to the robot arm 11. However, the present invention is not limited to them, and if an instrument to be set is a rotatable medical instrument necessary for being included in the sterilized zone, it is enough.

Furthermore, shapes of the adapter 47 for holding the medical instrument and the separator 46 which the adapter 47 is inserted in, are not limited to a cylindrical one. If they have shapes capable of attaching a medical instrument thereto rotatably around the prescribed axis, they are enough. Note that, it is necessary that the separator 46 has a shape of a hollow cylinder for inserting the adapter 47 therein, and it is necessary that the inner surface of the hollow cylinder is included in a sterilized zone.

Furthermore, the timing pulley 27 is adopted as the rotation mechanism of the robot arm 11, but the rotation mechanism is not limited to it and is enough if the rotation mechanism is one capable of transmitting the rotational force to the medical instrument via the separator 46 and the adapter 47. Furthermore, it is not absolutely necessary that the rotation mechanism is the robot arm 11. The present invention can be applied to all devices to be included in an unsterilized zone, each of which devices has a rotation mechanism for rotating the medical instrument.

Furthermore, in order to connect the drape 31 for separate a device having the rotation mechanism from a sterilized zone, in the above-described embodiment, a set of the outlet drape bearing 41 and the outlet drape bearing cover 42 and a set of the inlet drape bearing 45 and the inlet drape bearing cover 44 are adopted. However means for connecting the drape is not limited to them.

In other words, an adapter component set which the present invention is applied to, is enough by adopting the following construction, and may have various embodiments.

That is, the following adapter component set is enough. An adapter component set for connecting a medical instrument to a device that is included in an unsterilized zone and has a rotation mechanism for rotating the medical instrument, comprising:
an adapter for holding the medical instrument;
a separator having a shape of a hollow cylinder into which the adapter is inserted, at least an inner surface of the hollow cylinder being included in a sterilized zone, and an outer surface of the separator being configured to be capable of being connected to the rotation mechanism; and
connecting parts to which a drape is connected, the drape separating the device having the rotation mechanism from the sterilized zone.
Hereby, the unsterilized zone and the sterilized zone can be perfectly separated from each other.

Furthermore, it is preferable that a rotational force of the rotation mechanism is perfectly transmitted to the medical instrument. In order to achieve this, it is preferable that a first engagement parts for engaging the adapter and the separator, and second engagement parts for engaging the separator and the rotation mechanism are further comprised.

In this case, it is further preferable that an engagement strength of the first engagement parts is weaker than an engagement strength of the second engagement parts.

Since the adapter is disengaged from the separator when a pushing force over a predetermined force strength is applied to the medical instrument, a risk that the force pushes the medical instrument is reduced, and the unsterilized zone can be perfectly prevented from being exposed.

Further, the present invention is enough if the present invention has the following construction as a control system to carry out the pneumatic servo control for the pneumatic robot arm which holds a medical instrument and operates it, and may have various embodiments.

That is, the present invention is enough if the control system comprises:
a changeover device for switching a passive operation mode and an active operation mode, and
a controller which sets a pressure target based on a result of a position control with use of an error of a current position of the robot arm against a position target and carries out a pressure control based on the pressure target at the time of the active operation mode, and sets a pressure target necessary for a self weight compensation with use of a current position of the robot arm and carries out a pressure control based on the pressure target at the time of the passive operation mode.

Hereby, at the time of the passive operation mode, a user (doctor) can manually operate the robot arm set with the medical instrument with an intuitive feeling similar to that in a usual case where the user holds the medical instrument such as an endoscope or the like.

The changeover device is a device to be operated by the user's touch, and is positioned at a location where the user can touch it while operating the medical instrument.

The reason is that the operability by the user is greatly enhanced.

Note that, the series of processing according to the present invention to carry out the pneumatic servo control, can be carried out by each of software and hardware.

In a case where the series of processing is carried out by software, a program constituting the software can be installed into the computer or the like through a network or from a recording medium. The computer may be a computer incorporated with an exclusive hardware, or may be, for example, a general-purpose personal computer capable of performing various functions by installing various programs.

The recording medium including various programs for carrying out the series of processing according to the present invention may be a removable medium which is separated from the information processor body and distributed for supplying a program to a user, or may be a recording medium or the like incorporated in advance in the information processor body. The removable medium is constituted of, for example, a magnetic disk (including a floppy disk), an optical disk, a magnetooptical disk, or the like. Furthermore, as the recording medium incorporated in the information processor body, a hard disk or a ROM recorded with programs, or the like may be used.

Note that, in this description, steps describing programs recorded in a recording medium may include not only a series of processing to be carried out time-sequentially along the order but also a series of processing to be carried out in parallel or individually even if they are not necessarily time-sequentially processed.

Note that, in this description, the system means the whole device constituted by a plurality of devices and/or processing sections.

### DESCRIPTION OF THE SYMBOLS

- 1: Robot system
- 11: Robot arm
- 12: Surgical instrument
- 21: Rotation driving device
- 22: Connecting member
- 23: Parallel driving mechanism
- 24: Pneumatic cylinder
- 25: Rotation motor
- 26: Timing belt
- 27: Timing pulley
- 28: Adapter component set
- 31: Drape
- 41: Drape bearing
- 42: Outlet drape bearing cover
- 44: Inlet drape bearing cover
- 45: Inlet drape bearing
- 46: Separator
- 47: Adapter
- 101: Position detecting section
- 102: Pneumatic actuator
- 103: Changeover switch
- 104: Air pressure source
- 105: Servo valve
- 106: Pressure detecting section
- 107: Input/output section
- 108: Control section
- 201: Position target input section
- 202: Position controller
- 203: Pressure controller
- 204: Position detecting section
- 205: Self weight compensation controller

## Claims

1. An adapter component set for connecting a medical instrument to a device that is included in an unsterilized zone and has a rotation mechanism for rotating the medical instrument, comprising:
an adapter for holding the medical instrument;
a separator having a shape of a hollow cylinder into which the adapter is inserted, at least an inner surface of the separator being included in a sterilized zone, and an outer surface of the separator being configured to be capable of being connected to the rotation mechanism; and
connecting parts to which a drape is connected, the drape separating the device having the rotation mechanism from the sterilized zone.

2. The adapter component set according to claim 1, further comprising first engagement parts for engaging the adapter and the separator, and second engagement parts for engaging the separator and the rotation mechanism.

3. The adapter component set according to claim 2, wherein an engagement strength of the first engagement parts is weaker than an engagement strength of the second engagement parts.
